# EUROPÄISCHE PATENTSCHRIFT

(11) **EP 1 698 372 B1**
(45) Veröffentlichungstag und Bekanntmachung des Hinweises auf die Patenterteilung: **25.11.2009**
(21) Anmeldenummer: 06009032.1
(22) Anmeldetag: 24.10.2003
(51) Int. Cl.: A61N 1/32, C12M 1/42, H01C 1/14

(54) **Verfahren zur Herstellung eines elektrisch kontaktierbaren Bereichs auf einem dotierten Polymer und nach dem Verfahren herstellbare Elektroden**
Process for the formation of electrical contactable conductors on conductive polymer and electrodes obtainable therewith
Procédé de réalisation d'un élément conducteur sur un polymère conducteur et électrodes obtenus à partir dudit procédé

(43) Veröffentlichungstag der Anmeldung: 06.09.2006
(62) Teilanmeldung aus: 03024343.0
(73) Patentinhaber: Lonza Cologne AG, 50829 Köln (DE)
(72) Erfinder: Müller-Hartmann, Herbert, 50937 Köln (DE)

(56) Entgegenhaltungen:
- EP-A- 0 778 046
- EP-A- 1 100 295
- DE-A- 10 208 188
- DE-A1- 2 445 169
- US-A- 4 570 055
- US-A- 6 001 617
- US-A1- 2002 130 673

## Beschreibung

Die Erfindung betrifft ein Verfahren zur Herstellung zumindest eines elektrisch kontaktierbaren Bereichs auf einem mit einem leitfähigen Material dotierten Polymer, bei dem auf das Polymer ein Kontaktmaterial aufgebracht wird, welches bei 23°C einen geringeren spezifischen Widerstand als das Polymer aufweist. Die Erfindung betrifft ferner einen Formkörper aus mit leitfähigem Material dotiertem Polymer, mit zumindest einem kontaktierbaren Bereich, innerhalb dessen auf das Polymer ein Kontaktmaterial aufgebracht ist, welches bei 23°C einen geringeren spezifischen Widerstand als das Polymer aufweist.

Elektrisch leitfähige Polymere sind bekannt und erfreuen sich zunehmender Beliebtheit, insbesondere auch als Material zur Herstellung von Elektroden zur Erzeugung elektrischer Felder für unterschiedlichste Anwendungen.

Aus der US 4 570 055 ist ein Formkörper bekannt, bestehend aus einem mit leitfähigem Stoff dotiertem Polymer. Der Formkörper wird mittels ringförmiger Federelektroden kontaktiert und kann zur Verbesserung der Stromleitung an den Kontaktstellen der Elektroden mit Silberfarbe bestrichen sein.

Aus der EP 0 307 007 B1 ist beispielsweise eine Anordnung für elektrische Einrichtungen bekannt, die leitfähige Elemente mit unterschiedlichen spezifischen Widerständen aufweisen. Ein Widerstandselement aus einem leitfähigen Polymer, d. h. einem Gemisch aus einem organischen Polymer und einem leitfähigen Füllstoff, das bei 23°C einen relativ hohen spezifischen Widerstand von 1 - 500.000 Ohm·cm aufweist, wird dabei mit einer Kontaktschicht versehen, die aus einem leitfähigen Material besteht, dessen spezifischer Widerstand geringer als der des Widerstandselements ist, d.h. zwischen 2,5 x 10⁻⁵ und 1 x 10⁻³ Ohm·cm liegt. Die Kontaktschicht besteht ebenfalls aus einem leitfähigen Polymer, das mit einem Metall, beispielsweise Silber, oder einem kohlenstoffhaltigen Material, beispielsweise Graphit, dotiert ist. Die Kontaktschicht ist in Form von bandförmigen Elektroden, die fingerartig ineinander greifen, auf das Widerstandselement aufgebracht. Als Anschlusselemente sind Stromschienen vorgesehen, die aus einem gestreckten Metallnetz bestehen und um die Kontaktschicht bzw. die hieraus gebildeten Elektroden herumgefaltet sind. Dabei bilden die Randbereiche der Elektroden die Kontaktfläche. Zwar wird bei dieser Lösung der Eingangswiderstand der Widerstandsschicht durch das Aufbringen einer Kontaktschicht mit geringerem spezifischen Widerstand herabgesetzt, jedoch besteht diese Kontaktschicht ihrerseits aus einem dotierten Polymer und weist daher ebenfalls noch einen relativ hohen Eingangswiderstand auf. Dies liegt vor allem dann vor, wenn bei einem Spritzen der Kontaktschicht eine Entmischung an deren Oberfläche auftritt. Die Kontaktierung der Kontaktschicht erfolgt hier ferner über anliegende Stromschienen, d.h. gestreckte Metallnetze, und ist somit nicht mit einer punktförmigen Kontaktierung vergleichbar. Es gibt aber viele Anwendungen, bei denen aufgrund der spezifischen Erfordernisse oder der räumlichen Gegebenheiten eine punktförmige Kontaktierung, beispielsweise über Federkontakte, erforderlich ist. Eine punktförmige Kontaktierung der hier offenbarten Kontaktschicht würde beim Anlegen sehr hoher Spannungen aber zu einem Einbrennen der Anschlusselemente an den Kontaktsstellen führen.

Gerade im Bereich biologischer Anwendungen sind Elektroden aus leitfähigen Kunststoffen im Vergleich zu herkömmlichen Metallelektroden vorteilhaft, da aus den Metallelektroden bei einer elektrischen Entladung Metallionen freigesetzt werden können. Bei der Behandlung von lebenden Zellen, beispielsweise bei der Elektroporation oder der Elektrofusion, können die in die entsprechende Zellsuspension abgegebenen Metallionen entweder in geringer Konzentration zu einer unerwünschten Stimulierung der Zellen oder in höherer Konzentration zu einer toxischen Wirkung führen. So konnte beispielsweise bei der Verwendung von Aluminiumelektroden ein negativer Effekt auf die behandelten Zellen nachgewiesen werden, der durch freigesetzte Al³⁺-Ionen verursacht wird (Loomis-Husselbee et al., Biochem J 1991, 277 (Pt 3), 883 - 885). Darüber hinaus kann es bei der Verwendung von Metallelektroden zur Bildung von Metallhydroxyden oder Komplexen von Metallionen mit biologischen Makromolekülen kommen (Stapulionis, Bioelectrochem Bioenerg 1999, 48 (1), 249 - 254), was ebenfalls häufig unerwünscht ist.

Aus der DE 102 08 188 A1 sind Behälter mit Elektroden aus dotierten Polymeren bekannt. Bei den dotierten Polymeren handelt es sich um Polymere, denen leitfähige Stoffe wie beispielsweise Kohlefasern, Graphit, Russ oder Kohlenstoff-Nanotubes beigemischt sind. Solche dotierten Polymere weisen zwar im Vergleich zu intrinsisch leitenden Polymeren eine geringere Leitfähigkeit auf, haben aber den Vorteil, dass sie spritzfähig sind, d. h. im Spritzgussverfahren verarbeitet werden können. Solche dotierten Polymere sind also vielseitig anwendbar und ermöglichen dabei die kostengünstige Herstellung von Elektroden im Spritzgussverfahren. Ein Problem bei solchen Elektroden besteht aber darin, dass beim Spritzvorgang eine Entmischung stattfindet, so dass die Konzentration der leitfähigen Dotierung an der Oberfläche der Elektroden relativ gering ist. Entsprechende Elektroden haben daher einen sehr hohen Eingangswiderstand, was zur Folge hat, dass sehr hohe Spannungen angelegt werden müssen, um einen ausreichenden Stromfluss zu erreichen. Bei der üblichen punktförmigen Kontaktierung dieser Elektroden, beispielsweise durch Federkontakte, kommt es aber aufgrund der hohen angelegten Spannungen ebenfalls zu einem Einbrennen der Kontakte in die Elektrodenoberfläche, so dass diese unbrauchbar werden.

Aus der DE 101 16 211 A1 ist ferner eine Vorrichtung zur Fusion von lebenden Zellen im elektrischen Feld bekannt, bei der die Elektroden ebenfalls aus einem dotierten Kunststoff, d. h. einem Kunststoff, der mit Kohlenstoff gefüllt ist, hergestellt sind. Die Elektroden sind punktuell über Kontaktpunkte und entsprechende Leitungen mit einer Spannungsquelle verbunden. Auch hier besteht also der Nachteil, dass ein Einbrennen in die Oberfläche der Kunststoffelektroden eintritt, wenn höhere Spannungen angelegt werden sollten als die, die für eine Elektrodenfusion erforderlich sind. Um beispielsweise für besondere Anwendungen der Elektroporation ausreichende Feldstärken zu erreichen, müssten an die Elektroden wesentlich höhere Spannungen angelegt werden. So können beispielsweise für das Einbringen von biologisch aktiven Molekülen in den Zellkern lebender Zellen Feldstärken von 2-10 kV/cm erforderlich sein. Die zur Erreichung solcher Feldstärken erforderliche Spannung würde bei der punktförmigen Kontaktierung der bekannten Polymerelektroden auch hier zu einem Einbrennen der Kontaktstellen führen.

Es ist daher Aufgabe der Erfindung, die genannten Nachteile zu vermeiden und ein Verfahren der eingangs genannten Art bereitzustellen, welches eine effektive Reduzierung des Eingangswiderstands des Polymers in dem kontaktierbaren Bereich auf einfache und kostengünstige Weise ermöglicht, sowie einen Formkörper der eingangs genannten Art zu schaffen, der einen geringen Eingangswiderstand aufweist und einfach und kostengünstig herzustellen ist.

Erfindungsgemäß wird die Aufgabe durch ein Verfahren mit den Merkmales des Anspruchs 8 gelöst. Dabei ist das Kontaktmaterial eine Metallsuspension , die auf das Polymer aufgetragen wird. Durch das Auftragen der Metallsuspension, die einen geringeren spezifischen Widerstand als das Polymer aufweist, wird der Eingangswiderstand des dotierten Polymers effektiv herabgesetzt, wobei durch den engen Kontakt des Kontaktmaterials mit dem Polymer ein intensiver Kontakt zu der leitenden Dotierung des Polymers hergestellt wird. Die Dotierung des Polymers, die beispielsweise aus Kohlefasern und/oder Graphit bestehen kann, liegt in dem Polymer in einer bestimmten Konzentration vor, beispielsweise 75 Gew.-%, die gewährleistet, dass das dotierte Polymer eine ausreichende Leitfähigkeit aufweist. Um den Eingangswiderstand, insbesondere für Anwendungen, bei denen sehr hohe Feldstärken zwischen 2 und 10 kV/cm zwischen zwei Polymerelektroden erreicht werden müssen, effektiv zu verringern, muss ein Kontaktmaterial gewählt werden, das eine so ausreichende Verbindung zu der Dotierung des Polymers herstellt, dass die Temperatur an der Kontaktfläche zwischen Polymer und Kontaktmaterial während des Durchleitens von elektrischem Strom unterhalb des Erweichungspunktes des dotierten Polymers bleibt. Durch diese Maßnahmen kann ein mit leitfähigem Material dotiertes Polymer in dem kontaktierbaren Bereich auch punktuell, d.h. beispielsweise mit einem Draht oder einem Federkontakt, effektiv kontaktiert werden, ohne dass sich die Kontakte bei hohen angelegten Spannungen in die Oberfläche des Polymers einbrennen.

In vorteilhafter Ausgestaltung der Erfindung ist vorgesehen, dass die Metallsuspension eine kolloidale Silbersuspension ist, die auf das Polymer aufgetragen wird. Hierbei handelt es sich um ein besonders einfaches und kostengünstiges Verfahren, das bei speziellen Anwendungen vorteilhaft ist.

In besonders vorteilhafter Ausgestaltung der Erfindung ist ferner vorgesehen, dass die Oberfläche des Polymers vor dem Aufbringen des Kontaktmaterials zumindest teilweise durch mechanische und/oder chemische Behandlung vergrößert wird. Dabei kann die Oberfläche beispielsweise durch mechanische Verletzung aufgeraut werden. Diese Maßnahmen erleichtern das Aufbringen des Kontaktmaterials und sorgen darüber hinaus für eine noch engere Vernetzung der beiden Materialien.

Das Kontaktmaterial sollte vorzugsweise bei 23°C einen sehr geringen spezifischen Widerstand aufweisen, der beispielsweise unterhalb von 1 x 10⁻⁵ Ohm·cm liegt. Vorzugsweise sollte der spezifische Widerstand 1 x 10⁻⁶ bis 2 x 10⁻⁶ Ohm·cm betragen.

In vorteilhafter Ausgestaltung der Erfindung ist vorgesehen, dass das Kontaktmaterial über eine haftvermittelnde Schicht, die vorzugsweise einen geringen spezifischen Widerstand aufweist, auf das Polymer aufgebracht wird, um den Kontakt zwischen dem Kontaktmaterial und dem Polymer zu verbessern. Die haftvermittelnde Schicht kann dabei in vorteilhafter Ausgestaltung ein leitfähiges Material sein, beispielsweise ein elektrisch leitender Klebstoff oder Ähnliches.

Erfindungsgemäß wird die Aufgabe ferner durch eine Elektrode mit den Merkmales des Anspruchs 1 gelöst, wobei das Kontaktmaterial eine Metallsuspension ist, die mit dem leitfähigen Material in engem Kontakt steht. Ein solcher Formkörper weist einen deutlich verringerten Eingangswiderstand auf und kann problemlos auch punktuell, d.h. beispielsweise mit einem Draht oder einem Federkontakt, effektiv kontaktiert werden, ohne dass sich die Kontakte bei hohen angelegten Spannungen in die Oberfläche des Polymers einbrennen. Dabei ist der Kontakt zwischen dem Kontaktmaterial und der Dotierung des Polymers so intensiv, dass die Temperatur an der Kontaktfläche zwischen Kontaktmaterial und Polymer während des Durchleitens von elektrischem Strom unterhalb des Erweichungspunktes des dotierten Polymers liegt. Bei dem erfindungsgemäßen Formkörper ist dies dadurch gewährleistet, dass das Kontaktmaterial eine enge, d.h. sehr intensive, Verbindung zu der Dotierung des Polymers darstellt, die den Eingangswiderstand des Polymers effektiv verringert.

Das Kontaktmaterial kann in vorteilhafter Ausgestaltung der Erfindung eine kolloidale Silbersuspension sein, da dieses Material einen geringen spezifischen Widerstand aufweisen und leicht zu verarbeiten ist. Das Kontaktmaterial sollte einen spezifischen Widerstand bei 23°C unterhalb von 1 x 10⁻⁵ Ohm·cm, vorzugsweise von 1 x 10⁻⁶ bis 2 x 10⁻⁶ Ohm·cm, aufweisen.

Um den Eingangswiderstand des erfindungsgemäßen Formkörpers weiter zu verringern, kann zwischen dem Polymer und dem Kontaktmaterial eine kohlenstoffhaltige Schicht, vorzugsweise eine Graphitschicht, oder eine haftvermitteinde Schicht, die vorzugsweise einen geringen spezifischen Widerstand aufweist, angeordnet sein, welche jeweils einen intensiven Kontakt zu der leitfähigen Dotierung des Polymers vermitteln. Die haftvermittelnde Schicht kann dabei in vorteilhafter Ausgestaltung ein leitfähiges Material sein, beispielsweise ein elektrisch leitender Klebstoff oder Ähnliches.

Das Polymer ist vorzugsweise mit Kohlefasern, Graphit, Ruß und/oder Kohlenstoff-Nanotubes dotiert, wobei die Dotierung vorteilhafterweise in einer Konzentration von 50 bis 80 Gew.-% vorliegt. Das dotierte Polymer sollte dabei einen spezifischen Widerstand bei 23 °C von ungefähr 0,4 bis 1,0 Ohm·cm, vorzugsweise 0,46 Ohm·cm, aufweisen. Wird ein solches dotiertes Polymer mittels Spritzgießen verarbeitet, so verringert sich die Konzentration der Dotierung an der Oberfläche durch Entmischung. Der Oberflächenwiderstand ist daher relativ hoch, d. h. er liegt beispielsweise bei 2 bis 10 Ohm, insbesondere 8 bis 8,5 Ohm. Dieser Eingangswiderstand muss durch das dichte Aufbringen des Kontaktmaterials verringert werden, damit der Gesamtwiderstand des erfindungsgemäßen Formkörpers nicht zu hoch ist.

Das Polymer kann beispielsweise Polycarbonat, Polyetheretherketon, Polypropylen, Polyamid vorzugsweise Polyamid 6 oder Polyamid 66, Polyphenylensulfid oder ein Gemisch dieser Polymere sein oder eines oder mehrere dieser Polymere als Hauptbestandteil enthalten.

Der erfindungsgemäße Formkörper ist vorzugsweise als Elektrode oder ähnliches Bauteil ausgebildet, das zum Weiterleiten von elektrischem Strom benötigt wird. Der Formkörper kann auch, vorzugsweise in Form einer Elektrode, Bestandteil einer Küvette oder zumindest eines Reaktionsraums einer Multiwell-Platte sein, beispielsweise für die Elektroporation oder Elektrofusion von lebenden Zellen, insbesondere auch für HT-Anwendungen (HT = high throughput).

Tabelle 1 zeigt einen Vergleich der nach dem erfindungsgemäßen Verfahren hergestellten erfindungsgemäßen Formkörper mit entsprechenden Formkörpern ohne aufgebrachtes Kontaktmaterial. Verglichen wurde das Verhalten dotierter Polymerelektroden bei einer Kontaktierung mit Federkontakten aus Messing. Zunächst wurden Elektroden ohne jegliches Kontaktmaterial oder Zwischenschicht getestet. Bei einer Spannung von 1000 V brannten sich hierbei die Federkontakte deutlich in die Kontaktfläche der Elektroden ein. In einem weiteren Versuch wurden die Elektroden dann in eine Vorrichtung eingeschoben, in der der Kontakt nicht unmittelbar durch die Federkontakte erfolgte, sondern über eine Kupferfolie, die unter leichtem Druck auf die Kontaktfläche der Elektroden aufgebracht war. Auch hier konnte ein Einbrennen der Kupferfolie in die Kontaktfläche des dotierten Polymers beobachtet werden, wenn auch in schwächerer Ausprägung. Bei den verschiedenen Ausführungsformen der erfindungsgemäßen Formkörper bzw. nach dem erfindungsgemäßen Verfahren hergestellten Elektroden konnte unter gleichen Bedingungen kein Einbrennen der Federkontakte oder des Kontaktmaterials in die Kontaktfläche des dotierten Polymers beobachtet werden. Durch das dichte Aufbringen des jeweiligen Kontaktmaterials auf das dotierte Polymer kann also ein Einbrennen eines verwendeten Anschlusselements auch bei einer hohen angelegten Spannung von 1000 V sicher vermieden werden.

**Tabelle 1: Verhindern des Einbrennens in die Oberfläche von Formkörpern in Form von Elektroden aus dotiertem Polymer (Polyamid 6 mit Kohlefasern und Graphit)**

| **Kontaktierung** | **Angelegte Gleichspannung; geflossene Ladung** | **Einbrennen; Oberflächenbeschädigung** |
|---|---|---|
| Federkontakte (Messing) | 1000 V; 5 mC | Ja, deutlich |
| Kupferfolienadapter (ca. 20 mN/mm²) | 1000 V; 5 mC | Ja, schwach |
| Kupferplatten (ca. 2 N/mm²) * | 1000 V; 5 mC | Nein |
| Kupferplatten + Graphit (ca. 2 N/mm²) ** | 1000 V; 5 mC | Nein |
| 55 µm Kupferfolie, heiß geprägt, 100 mm² | 1000 V; 5 mC | Nein |
| 55 µm Kupferfolie, heiß geprägt, 8 mm² | 1000 V; 5 mC | Nein |

| | | |
|---|---|---|
| * Auflagefläche ca. 100 mm² ** Graphit: Dichte 1 g/cm³, 1 mm Stärke, 100 mm² Auflagefläche auf Polymer | | |

Die Erfindung wird im Weiteren anhand der Figuren beispielhaft näher erläutert.

Es zeigt:
- Figur 1: einen Querschnitt durch eine Polymerküvette, die mit nach dem erfindungsgemäßen Verfahren hergestellten Elektroden ausgestattet ist,
- Figur 2: Balkendiagramme der Stromstärke und des Widerstandes, die einen Vergleich der Leitfähigkeit zwischen Elektroden mit und ohne aufgebrachtes Kontaktmaterial zeigen, wobei die verwendeten Polymerküvetten mit Elektroden aus einem dotierten Polymer (Polyamid 6 mit Kohlefasern und Graphit) ausgestattet sind und das Kontaktmaterial eine mittels Heißprägen aufgebrachte Kupferfolie ist, Stärke der Kupferfolie: 55 µm, Spaltbreite der Küvetten: 1,5 mm, Volumen der in den Küvetten befindlichen Elektrolytlösung: 100 µl, angelegte Spannung: 1000 V Gleichspannung,
A: Elektrolytlösung mit spezifischer Leitfähigkeit von 17,02 mS/cm, n=6
B: Elektrolytlösung mit spezifischer Leitfähigkeit von 17,02 mS/cm, n = 2,
- Figur 3: Balkendiagramme der Stromstärke und des Widerstandes eines Vergleichs der Leitfähigkeit von Polymerküvetten mit auf unterschiedliche Kontaktflächen des dotierten Polymers nach dem erfindungsgemäßen Verfahren aufgebrachter Kupferfolie, breit = 100 mm² und schmal = 18 mm², Spaltbreite der Küvetten = 1,5 mm, Volumen der Elektrolytlösung = 100 µl, spezifische Leitfähigkeit der Elektrolytlösung = 12,75 mS/cm, Stärke der Kupferfolie = 55 µm, graue Balken = 1000 V Gleichspannung, schwarze Balken = 500 V Gleichspannung, n = 2,
- Figur 4: eine perspektivische Ansicht einer Küvette mit erfindungsgemäßen Formkörpern und
- Figur 5: eine perspektivische Ansicht der Küvette gemäß Figur 4 mit einer weiteren Ausführungsform erfindungsgemäßer Formkörper.

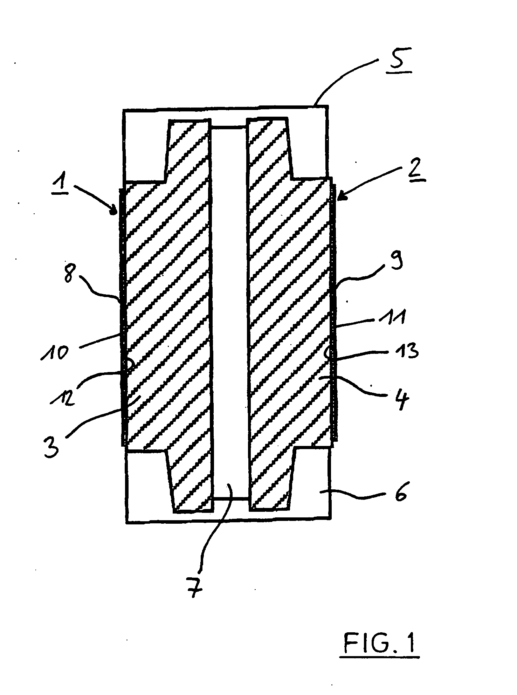

Figur 1 zeigt einen Querschnitt durch zwei erfindungsgemäße Formkörper 1, 2, die in diesem Ausführungsbeispiel als Elektroden 3, 4 einer Küvette 5 ausgebildet sind. Die Küvette 5 besteht aus einem Rahmen 6, der aus einem spritzfähigen Polymer hergestellt ist und der in seinem Bodenbereich zwei gegenüberliegend angeordnete Elektroden 3, 4 aufweist. Die Elektroden 3, 4 bestehen ebenfalls aus einem spritzfähigen Polymer, das mit leitfähigen Stoffen, beispielsweise Kohlefasern und/oder Graphit, dotiert ist. Die parallel gegenüberliegend angeordneten Elektroden 3, 4 schließen einen spaltförmigen Innenraum 7 ein, welcher der Aufnahme einer Flüssigkeit, beispielsweise einer Elektrolytlösung, dient. Beim Anlegen einer elektrischen Spannung an die beiden Elektroden 3, 4 fließt ein elektrischer Strom durch die Flüssigkeit bzw. Elektrolytlösung im Innenraum 7. Beispielsweise können in der Elektrolytlösung lebende Zellen suspendiert sein, in die in der Elektrolytlösung gelöste biologisch aktive Moleküle, beispielsweise Nukleinsäuren oder Proteine, mit Hilfe des durch den Innenraum 7 fließenden elektrischen Stroms eingebracht werden können. Zum Anlegen einer Spannung müssen die Elektroden 3, 4 an ihren Außenseiten 8, 9 mittels eines geeigneten Anschlusselementes, beispielsweise eines Federkontakts, kontaktiert werden. Um ein Einbrennen des Anschlusselements, insbesondere beim Anlegen sehr hoher Spannungen, zu vermeiden und den Eingangswiderstand des Polymers zu reduzieren, ist auf die beiden Außenseiten 8, 9 der Elektroden 3, 4 jeweils ein Kontaktmaterial 10, 11 mit hoher Leitfähigkeit dicht aufgebracht. Das Kontaktmaterial 10, 11 bedeckt dabei im vorliegenden Ausführungsbeispiel jeweils die gesamte jeweils zur Verfügung stehende Kontaktfläche 12, 13 der Elektroden 3, 4. Das Kontaktmaterial 10, 11 sollte jedenfalls einen spezifischen Widerstand bei 23°C von weniger als 1 x 10⁻⁵ Ohm·cm aufweisen. Das Kontaktmaterial 10, 11 muss ferner in jedem Fall dicht auf die Kontaktflächen 12, 13 aufgebracht werden, um den hohen Eingangswiderstand der Elektroden 3, 4 effektiv zu reduzieren. Zu diesem Zweck kann das Kontaktmaterial 10, 11 mittels einer haftvermittelnden Schicht auf die Kontaktflächen 12, 13 aufgebracht, beispielsweise aufgeklebt, werden. Durch das sehr dichte Aufbringen des Kontaktmaterials 10, 11 auf die Kontaktflächen 12, 13 der Elektroden 3, 4 kommt dieses mit der Dotierung der Elektroden 3, 4, d. h. dem leitfähigen Material innerhalb des Polymers, in engen, d.h. besonders intensiven, Kontakt, so dass das Kontaktmaterial 10, 11 praktisch eine Verbindung zwischen dem hier nicht dargestellten Anschlusselement, beispielsweise einem Federkontakt, und dem leitfähigen Material innerhalb der Elektroden 3, 4 herstellt. Auf diese Weise wird der Eingangswiderstand der Elektroden 3, 4 deutlich verringert, so dass bei hohen Spannungen weniger Energie in Form von Wärme an den Kontaktflächen 12, 13, d.h. dem kontaktierbaren Bereich, frei wird. Die Temperatur an den Kontaktflächen 12, 13 bleibt durch diese Maßnahme unterhalb des Schmelz-oder Erweichungspunktes des Polymers, aus dem die Elektroden 3, 4 hergestellt sind, so dass ein Einbrennen des Anschlusselementes und des Kontaktmaterials verhindert wird. Die nach dem erfindungsgemäßen Verfahren hergestellten erfindungsgemäßen Formkörper 1, 2 können also vorteilhafterweise auch für Anwendungen verwendet werden, bei denen mit hohen Spannungen gearbeitet wird und eine punktförmige Kontaktierung verwendet werden soll bzw. muss.

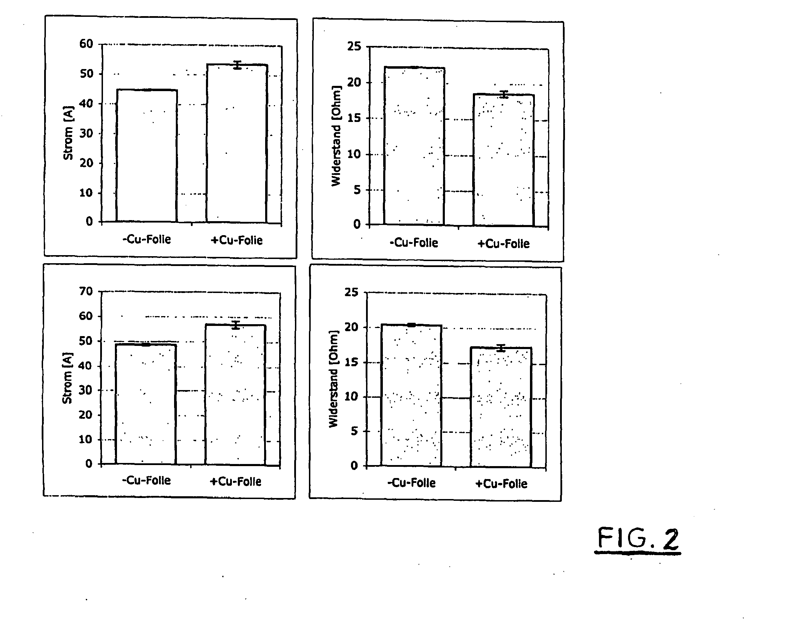

Figur 2 zeigt Vergleiche der Leitfähigkeiten von Polymerküvetten gemäß Figur 1, die mit Elektroden mit mittels Heißprägen aufgebrachten Kupferfolien ausgestattet sind, im Vergleich zu entsprechenden Küvetten mit Elektroden aus dotiertem Polymer ohne Kontaktmaterial bzw. aufgeprägte Kupferfolie. Aus den gezeigten Balkendiagrammen ist anhand des gesamten Stromflusses und des gemessenen Gesamtwiderstandes ersichtlich, dass das auf die Elektroden aufgebrachte Kontaktmaterial die Leitfähigkeit der Elektroden erhöht. In allen Fällen konnte mit Kontaktmaterial (Cu-Folie) ein erhöhter Stromfluss und ein verringerter Gesamtwiderstand gemessen werden. Da der Unterschied zwischen den verglichenen Küvetten ausschließlich in dem auf die Kontaktflächen der Elektroden aufgebrachten Kontaktmaterial besteht, zeigen diese Versuche, dass durch das Kontaktmaterial der Eingangswiderstand der Elektroden verringert werden konnte.

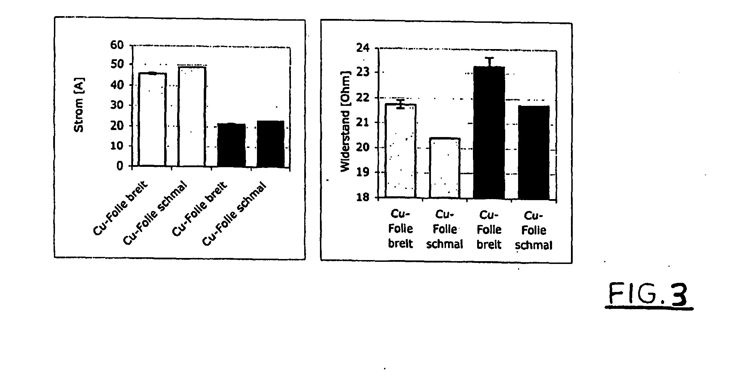

Figur 3 zeigt einen Vergleich zwischen Polymerküvetten gemäß Figur 1, bei denen das Kontaktmaterial, hier eine Kupferfolie, auf unterschiedlich große Kontaktflächen des Polymers dicht aufgebracht ist. Der Vergleich wurde bei unterschiedlichen Spannungen durchgeführt. Überraschenderweise hat sich bei diesen Versuchen herausgestellt, dass eine Verringerung der Kontaktfläche mit einer deutlicheren Reduzierung des Widerstands einhergeht. Die Erklärung für dieses Phänomen liegt möglicherweise darin, dass sich der Anpressdruck bei den schmalen Kupferfolien auf eine geringere Fläche verteilt und somit ein besserer Kontakt zu dem leitfähigen Material innerhalb des Polymers hergestellt wird. Dieser Effekt überwiegt dann möglicherweise den vermuteten negativen Effekt einer geringeren Fläche.

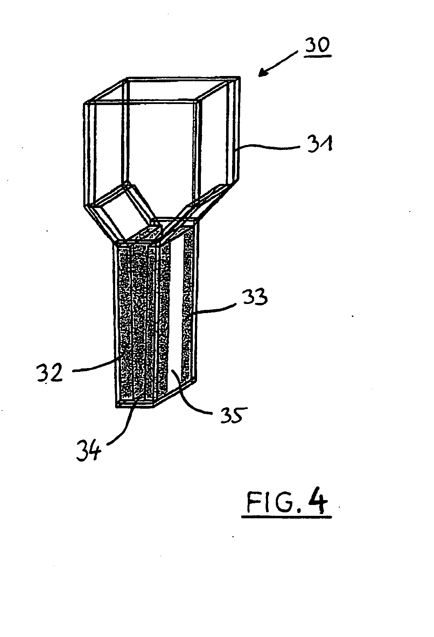

Figur 4 zeigt eine perspektivische Ansicht einer Küvette 30, die aus einem Grundkörper 31 und zwei erfindungsgemäßen Formkörpern 32, 33 besteht. Die Formkörper 32, 33 sind im unteren, verengten Bereich des Grundkörpers 31 angeordnet. Die parallel angeordneten Formkörper 32, 33 schließen dabei einen Spalt 34 ein, der der Aufnahme beispielsweise einer Zellsuspension dient. Wie beim Formkörper 33 ersichtlich, ist auf die Formkörper 32, 33 ein Kontaktmaterial 35 streifenförmig aufgebracht. Die Formkörper 32, 33 können beispielsweise aus einem mit Kohlefasern und Graphit dotiertem Polymer, beispielsweise Polyamid 6 oder Polyamid 66, bestehen. Beim Anlegen einer elektrischen Spannung an das Kontaktmaterial 35 wirken die Formkörper 32, 33 als Elektroden, so dass in dem Spalt 34 ein elektrisches Feld entsteht. Mit Hilfe dieses elektrischen Feldes können beispielsweise biologisch aktive Moleküle mittels Elektroporation in die Zellen eingebracht oder mittels Elektrofusion fusioniert werden.

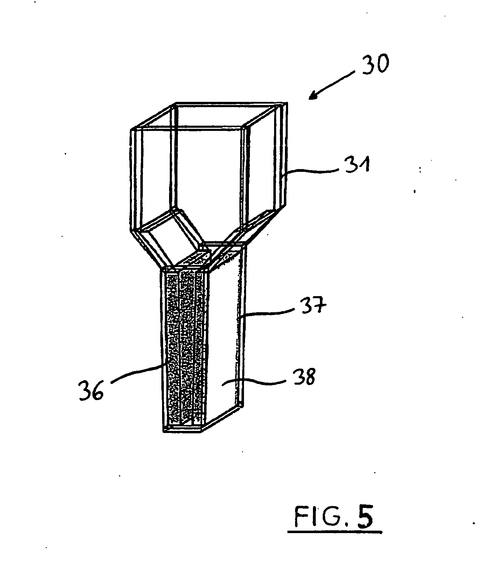

Figur 5 zeigt eine perspektivische Ansicht der Küvette 30 gemäß Figur 4, die ebenfalls im unteren Bereich zwei Elektroden 36, 37 aufweist. Im Gegensatz zu den Formkörpern 32, 33 gemäß Figur 4 ist bei den Elektroden 36, 37 ein Kontaktmaterial 38 ganzflächig auf das dotierte Polymer aufgebracht. Dies hat den Vorteil, dass für die Kontaktierung eine größere Fläche zur Verfügung steht.

### Bezugszeichenliste:

- 1: Formkörper
- 2: Formkörper
- 3: Elektrode
- 4: Elektrode
- 5: Küvette
- 6: Rahmen
- 7: Innenraum
- 8: Außenseite
- 9: Außenseite
- 10: Kontaktmaterial
- 11: Kontaktmaterial
- 12: Kontaktfläche
- 13: Kontaktfläche
- 30: Küvette
- 31: Grundkörper
- 32: Formkörper
- 33: Formkörper
- 34: Spalt
- 35: Kontaktmaterial
- 36: Elektrode
- 37: Elektrode
- 38: Kontaktmaterial

## Patentansprüche

1. Elektrode (3, 4, 36, 37), insbesondere als Bestandteil einer Küvette oder eines Reaktionsraums einer Multiwell-Platte, zumindest bestehend aus mit leitfähigem Material dotiertem Polymer, die zumindest einen kontaktierbaren Bereich aufweist, innerhalb dessen auf das Polymer ein Kontaktmaterial (10, 11, 35, 38) aufgebracht ist, welches bei 23°C einen geringeren spezifischen Widerstand als das Polymer aufweist und mittels eines Anschlusselements kontaktierbar ist, **dadurch gekennzeichnet, dass** das Kontaktmaterial (10, 11, 35, 38) eine Metallsuspension ist.

2. Elektrode nach Anspruch 1, **dadurch gekenntzeichnet, dass** die Metallsuspension eine kolloidale Silbersuspension ist.

3. Elektrode nach Anspruch 1 oder 2, **dadurch gekennzeichnet, dass** das Kontaktmaterial einen spezifischen Widerstand bei 23°C unterhalb von 1 x 10⁻⁵ Ohm·cm, vorzugsweise von 1 x 10⁻⁶ bis 2 x 10⁻⁹ Ohm·cm, aufweist.

4. Elektrode nach einem der Ansprüche 1 bis 3, **dadurch gekennzeichnet, dass** zwischen dem Polymer und dem Kontaktmaterial (10, 11, 35, 38) eine kohlenstoffhaltige Schicht, vorzugsweise eine Graphitschicht, oder eine haftvermittelnde Schicht, die vorzugsweise einen geringen spezifischen Widerstand aufweist, angeordnet ist.

5. Elektrode nach einem der Ansprüche 1 bis 4, **dadurch gekennzeichnet, dass** das Polymer mit Kohlefasern, Graphit, Ruß und/oder Kohlenstoff-Nanotubes dotiert ist und dass die Dotierung in einer Konzentration von 50 bis 80 Gew.-% vorliegt.

6. Elektrode nach einem der Ansprüche 1 bis 5, **dadurch gekennzeichnet, dass** das Polymer Polycarbonat, Polyetheretherketon, Polypropylen, Polyamid vorzugsweise Polyamid 6 oder Polyamid 66, Polyphenylensulfid oder ein Gemisch dieser Polymere ist oder eines oder mehrere dieser Polymere als Hauptbestandteil enthält.

7. Küvette oder Reaktionsraum einer Multiwell-Platte mit mindestens einer Elektrode (3, 4, 36, 37) nach einem der Ansprüche 1 bis 6.

8. Verfahren zur Herstellung zumindest eines elektrisch kontaktierbaren Bereichs auf einer zumindest aus einem mit einem leitfähigen Material dotierten Polymer bestehenden Elektrode nach einem der Ansprüche 1 bis 6, bei dem auf das Polymer ein Kontaktmaterial (10, 11, 35, 38) aufgebracht wird, welches bei 23°C einen geringeren spezifischen Widerstand als das Polymer aufweist, **dadurch gekennzeichnet, dass** als Kontaktmaterial (10, 11, 35, 38) eine Metallsuspension auf das Polymer aufgetragen und die Oberfläche des Polymers vor dem Auftragen des Kontaktmaterials (10, 11, 35, 38) zumindest teilweise durch mechanische und/oder chemische Behandlung vergrößert wird.

9. Verfahren nach Anspruch 8, **dadurch gekennzeichnet, dass** die Metallsuspension eine kolloidale Silbersuspension ist.

10. Verfahren nach Anspruch 8 oder 9, **dadurch gekennzeichnet, dass** die Oberfläche des Polymers durch mechanische Verletzung aufgeraut wird.

11. Verfahren nach einem der Ansprüche 8 bis 10, **dadurch gekennzeichnet, dass** das Kontaktmaterial (10, 11, 35, 38) einen spezifischen Widerstand bei 23°C unterhalb von 1 x 10⁻⁵ Ohm·cm, vorzugsweise von 1 x 10⁻⁶ bis 2 x 10⁻⁶ Ohm·cm, aufweist.

12. Verfahren nach einem der Ansprüche 8 bis 11, **dadurch gekennzeichnet, dass** das Kontaktmaterial (10, 11, 35, 38) über eine haftvermittelnde Schicht, die vorzugsweise einen geringen spezifischen Widerstand aufweist, auf das Polymer aufgebracht wird.

## Claims

1. An electrode (3, 4, 36, 37), particularly as part of a vessel or of a reaction space in a multi-well plate, at least comprising a polymer doped with conductive material, which has at least one contactable area, within which a contact material is applied to the polymer (10, 11, 35, 38), which has a lower specific resistance than the polymer at 23 °C and is contactable by means of a connection element, **characterised in that** the contact material (10, 11, 35, 38) is a metal suspension.

2. The electrode according to claim 1, **characterised in that** the metal suspension is a colloidal silver suspension.

3. The electrode according to claim 1 or 2, **characterised in that** the contact material has a specific resistance at 23 °C of below 1 x 10⁻⁵ Ohm·cm, preferably 1 x 10⁻⁶ to 2 x 10⁻⁶ Ohm·cm.

4. The electrode according to one of the claims 1 to 3, **characterised in that** there is a carbonaceous layer disposed between the polymer and the contact material (10, 11, 35, 38), preferably a graphite layer, or an adhesion-enhancing layer, which preferably has a low specific resistance.

5. The electrode according to one of the claims 1 to 4, **characterised in that** the polymer is doped with carbon fibres, graphite, soot and/or carbon nanotubes and that the doping is at a concentration of 50 to 80 % by weight.

6. The electrode according to one of the claims 1 to 5, **characterised in that** the polymer consists of polycarbonate,polyetheretherketone, polypropylene, polyamide, preferably polyamide 6 or polyamide 66, polyphenylene sulphide or a mixture of these polymers or that the polymer contains one or more of these polymers as its main constituent.

7. A vessel or reaction space in a multi-well plate with at least one electrode (3, 4, 36, 37) according to one of the claims 1 to 6.

8. A method for generating at least one electrically contactable area on an electrode, said electrode at least comprising a polymer doped with a conductive material according to one of the claims 1 to 6, wherein a contact material (10, 11, 35, 38) having a lower specific resistance at 23 °C than the polymer is applied to said polymer, **characterised in that** the contact material (10, 11, 35, 38) that is applied to the polymer is a metal suspension and that the surface of the polymer is enlarged at least partly by mechanical and/or chemical treatment before the contact material (10, 11, 35, 38) is applied.

9. The method according to claim 8, **characterised in that** the metal suspension is a colloidal silver suspension.

10. The method according to claim 8 or 9, **characterised in that** the polymer surface is roughened by mechanical damage.

11. The method according to one of the claims 8 to 10, **characterised in that** the contact material (10, 11, 35, 38) has a specific resistance at 23 °C below 1 x 10⁻⁵ Ohm·cm, preferably of 1 x 10⁻⁶ to 2 x 10⁻⁶ Ohm·cm.

12. The method according to one of the claims 8 to 11, **characterised in that** the contact material (10, 11, 35, 38) is applied to the polymer via an adhesion-mediating layer, which preferably has a low specific resistance.

## Revendications

1. Electrode (3, 4, 36, 37), en particulier comme composant d'une cuvette ou d'un espace de réaction d'une plaque multi-well, composée au moins de polymère dopé avec du matériau conducteur, laquelle plaque présente au moins une zone pouvant établir un contact, à l'intérieur de laquelle est appliqué sur le polymère un matériau de contact (10, 11, 35, 38), qui présente à 23°C une résistance spécifique plus faible que le polymère et peut être mis en contact au moyen d'un élément de raccordement, **caractérisée en ce que** le matériau de contact (10, 11, 35, 38) est une suspension de métal.

2. Electrode selon la revendication 1, **caractérisée en ce que** la suspension de métal est une suspension d'argent colloïdale.

3. Electrode selon la revendication 1 ou 2,
**caractérisée en ce que** le matériau de contact présente une résistance spécifique à 23°C inférieure 1 x 10⁻⁵ Ohm·cm, de préférence à 1 x 10⁻⁶ jusqu'à 2 x 10⁻⁶ Ohm·cm.

4. Electrode selon l'une quelconque des revendications 1 à 3, **caractérisée en ce que** entre le polymère et le matériau de contact (10, 11, 35, 38) est disposée une couche à base de carbone, de préférence une couche de graphite ou une couche adhérente, qui présente de préférence une faible résistance spécifique.

5. Electrode selon l'une quelconque des revendications 1 ou 4, **caractérisée en ce que** le polymère est dopé avec des fibres de carbone, du graphite, de la suie et/ou des nanotubes de carbone et **en ce que** le dopage est présent dans une concentration de 50 à 80 % en poids.

6. Electrode selon l'une quelconque des revendications 1 à 5, **caractérisée en ce que** le polymère est du polycarbonate, du polyétheréthercétone, du polypropylène, du polyamide, de préférence du polyamide 6 ou du polyamide 66, du sulfure de polyphénylène ou un mélange de ces polymères ou contient un ou plusieurs de ces polymères comme composant principal.

7. Cuvette ou espace de réaction d'une plaque Multiwell comprenant au moins une électrode (3, 4, 36, 37) selon l'une quelconque des revendications 1 à 6.

8. Procédé pour fabriquer au moins une zone pouvant être mise en contact électrique sur une électrode composée au moins d'un polymère dopé avec un matériau conducteur selon l'une quelconque des revendications 1 à 6, dans lequel un matériau de contact (10, 11, 35, 38) est appliqué sur le polymère, lequel matériau présente à 23°C une résistance spécifique plus faible que le polymère, **caractérisé en ce qu'**une suspension de métal est appliquée sur le polymère comme matériau de contact (10, 11, 35, 38) et la surface du polymère est agrandie au moins partiellement par traitement mécanique et/ou chimique avant l'application du matériau de contact (10, 11, 35, 38).

9. Procédé selon la revendication 8, **caractérisé en ce que** la suspension de métal est une suspension d'argent colloïdale.

10. Procédé selon la revendication 8 ou 9, **caractérisé en ce que** la surface du polymère est rendue rugueuse par blessure mécanique.

11. Procédé selon l'une quelconque des revendications 8 à 10, **caractérisé en ce que** le matériau de contact (10, 11, 35, 38) présente à 23°C une résistance spécifique inférieure à 1 x 10⁻⁵ Ohm·cm, de préférence à 1 x 10⁻⁶ jusqu'à 2 x 10⁻⁶ Ohm.cm.

12. Procédé selon l'une quelconque des revendications 8 à 11, **caractérisé en ce que** le matériau de contact (10, 11, 35, 38) est appliqué sur le polymère au moyen d'une couche adhérente, qui présente de préférence une faible résistance spécifique.
